# EUROPEAN PATENT APPLICATION

(11) **EP 3 026 047 A1**
(43) Date of publication of application: **01.06.2016**
(21) Application number: 14829555.3
(22) Date of filing: 22.07.2014
(51) Int. Cl.: C07D 311/70, C07D 491/052, C07B 61/00

(54) **METHOD FOR PRODUCING HETEROCYCLIC COMPOUND**

(30) Priority: 25.07.2013 JP 2013154943
(71) Applicant: Nissan Chemical Industries, Ltd., Chiyoda-ku Tokyo 101-0054 (JP)
(72) Inventor: NAGAYA, Akihiro, Funabashi-shi Chiba 274-8507 (JP); YOSHINO, Madoka, Funabashi-shi Chiba 274-8507 (JP); YOSHINO, Hironobu, Funabashi-shi Chiba 274-8507 (JP)
(74) Representative: Hoffmann Eitle
(86) International application number: PCT/JP2014/069351
(87) International publication number: WO 2015/012271

(57) **Abstract**

There is provided a method for producing with a high efficiency a heterocyclic compound that is useful as a raw material for pharamaceuticals. A novel production method for producing 2,2,7,9-tetramethyl-2H-pyrano[2,3-g]quinoline (compound (6)) that has a quinoline ring and a chromene ring using N-(3-acetyl-4-hydroxyphenyl)butylamide (compound (1')) that is commercially available as a raw material for pharamaceuticals or the like as a starting raw material; and a method for purifying compound (6), characterized by purifying by subjecting it to conversion into a salt form.

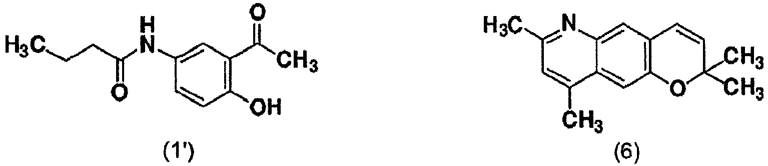

## Description

### TECHNICAL FIELD

The present invention relates to a method for producing a heterocyclic compound, particularly 2,2,7,9-tetramethyl-2H-pyrano[2,3-g]quinoline of Formula (6).

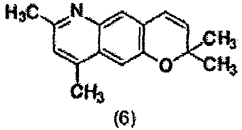

### BACKGROUND ART

(3R*,4S*)-7-hydroxymethyl-2,2,9-trimethyl-4-(phenethylamino)-3,4-dihydro-2H-p yrano[2,3-g]quinolin-3-ol (compound (9)) has an anti-arrhythmic action, and has been known to have a probability of use as a pharmaceutical (e.g., see Patent Document 1). 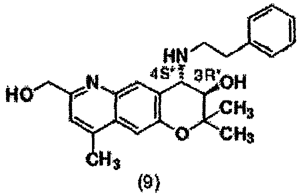 (* is a relative configuration.)

As a method for producing the compound (9), a method for producing 2,2-dimethyl-2H-chromen-6-amine (compound (5)) and 2,2,7,9-tetramethyl-2H-pyrano[2,3-g]quinoline (compound (6)) shown below as production intermediates has been known.

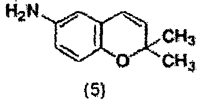

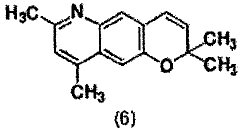

For example, the compound (6) can be produced by the following procedures. First, 1-fluoro-4-nitrobenzene (compound (10)) is reacted with 2-methyl-3-butyn-2-ol (compound (11)) to produce 1-((2-methyl-3-butyn-2-yl)oxy)-4-nitrobenzene (compound (12)), as shown below (e.g., see Patent Document 2). Subsequently, the compound (12) is heated and converted into 2,2,-dimethyl-6-nitro-2H-chromene (compound (13)) (e.g., see Non-Patent Document 1 and Non-Patent Document 2). A nitro group of the obtained compound (13) is reduced in the presence of a catalyst to obtain 2,2-dimethyl-2H-chromen-6-amine (compound (5)) (e.g., see Patent Document 3). Further, the compound (5) is reacted with 3-penten-2-one (compound (8)) to obtain the compound (6) (to perform quinoline cyclization) (e.g., see Patent Document 1 and Non-Patent Document 3).

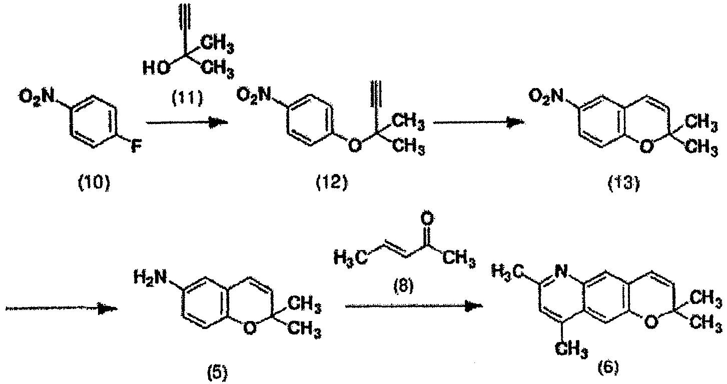

### Prior Art Documents

### Patent Documents

Patent Document 1: International Publication No. WO 2005/090357 Pamphlet
Patent Document 2: International Publication No. WO 2004/035520 Pamphlet
Patent Document 3: International Publication No. WO 2004/020428 Pamphlet

### Non-Patent Documents

Non-Patent Document 1: Journal of Med. chem., 34(5), 1570, 1991
Non-Patent Document 2: Synthesis, (6), 707, 1995
Non-Patent Document 3: Tetrahedron, 53(17), 6041, 1997

### SUMMARY OF THE INVENTION

### Problem to be Solved by the Invention

However, for industrial applications, the aforementioned method has many problems such as requiring a long time to react the compound (10) with the compound (11) to obtain the compound (13), and having a disadvantage in terms of production cost by use of an expensive precious metal catalyst such as platinum and palladium during reduction of a nitro group of the compound (13). A novel method for producing a heterocyclic compound capable of solving the problems has been desired.

That is, it is an object of the present invention to provide a method for producing 2,2,7,9-tetramethyl-2H-pyrano[2,3-g]quinoline as a heterocyclic compound.

### Means for Solving the Problem

The inventors of the present invention have intensively studied, and found a novel method for highly efficiently synthesizing 2,2-dimethyl-2H-chromen-6-amine (compound (5)) that is a production intermediate of 2,2,7,9-tetramethyl-2H-pyrano[2,3-g]quinoline (compound (6)). Further, the inventors have found a production method capable of highly efficiently obtaining a high-purity compound (6) relating to a step of obtaining the compound (6) from the compound (5). Thus, the present invention has been accomplished.

That is, the present invention is characterized by the following items (I) to (VIII).
(I) A method for producing 2,2-dimethyl-2H-chromen-6-amine comprising the following steps (a) to (d) (in the following formulae (1) to (4), R¹ is a C₁₋₆ alkyl group):
   (a) reacting a compound of Formula (1): 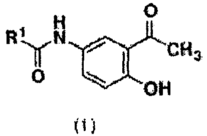 with acetone to obtain a chromanone ring derivative of Formula (2): 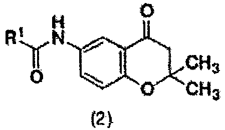
   (b) reducing the obtained chromanone ring derivative to obtain an alcohol derivative of Formula (3): 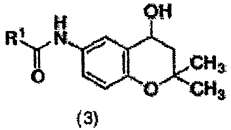
   (c) dehydrating the obtained alcohol derivative to obtain a chromene ring derivative of Formula (4): 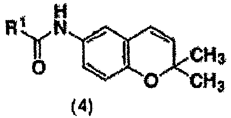 and
   (d) hydrolyzing the obtained chromene ring derivative to obtain 2,2-dimethyl-2H-chromen-6-amine of Formula (5). 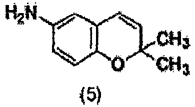
(II) The method according to (I), wherein R¹ is a n-propyl group.
(III) A method for producing 2,2,7,9-tetramethyl-2H-pyrano[2,3-g]quinoline comprising the following steps (e) and (f):
   (e) reacting 2,2-dimethyl-2H-chromen-6-amine of Formula (5): 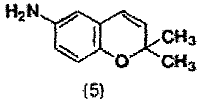 with 3-penten-2-one for cyclization, to obtain 2,2,7,9-tetramethyl-2H-pyrano[2,3-g]quinoline of Formula (6): 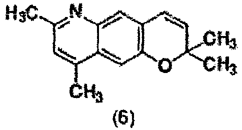
   and (f) converting the obtained 2,2,7,9-tetramethyl-2H-pyrano[2,3-g]quinoline into a salt form, to obtain the salt of 2,2,7,9-tetramethyl-2H-pyrano[2,3-g]quinoline as a crystal, and neutralizing the crystal of the salt to obtain 2,2,7,9-tetramethyl-2H-pyrano[2,3-g]quinoline having higher purity.
(IV) The method according to (III), wherein in the step (f), maleic acid is used for formation of a salt to produce 2,2,7,9-tetramethyl-2H-pyrano[2,3-g]quinoline malate of Formula (7). 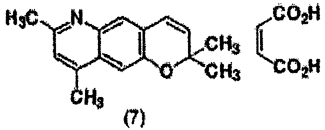
(V) A method for producing 2,2,7,9-tetramethyl-2H-pyrano[2,3-g]quinoline comprising the following steps (a) to (f) (in the following formulae (1) to (4), R¹ is a C₁₋₆ alkyl group):
   (a) reacting a compound of Formula (1): 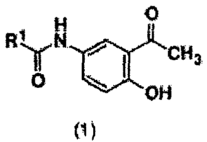 with acetone to obtain a chromanone ring derivative of Formula (2): 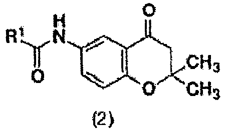
   (b) reducing the obtained chromanone ring derivative to obtain an alcohol derivative of Formula (3): 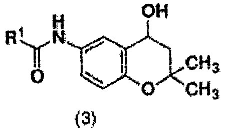
   (c) dehydrating the obtained alcohol derivative to obtain a chromene ring derivative of Formula (4): 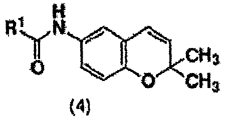
   (d) hydrolyzing the obtained chromene ring derivative to obtain 2,2-dimethyl-2H-chromen-6-amine of Formula (5): 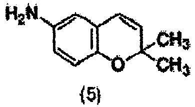
   (e) reacting the obtained 2,2-dimethyl-2H-chromen-6-amine with 3-penten-2-one for cyclization, to obtain 2,2,7,9-tetramethyl-2H-pyrano[2,3-g]quinoline of Formula (6): 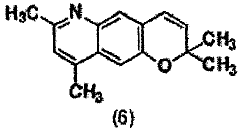 and
   (f) converting the obtained 2,2,7,9-tetramethyl-2H-pyrano[2,3-g]quinoline into a salt form, to obtain the salt of 2,2,7,9-tetramethyl-2H-pyrano[2,3-g]quinoline as a crystal, and neutralizing the crystal of the salt to obtain 2,2,7,9-tetramethyl-2H-pyrano[2,3-g]quinoline having higher purity.
(VI) A compound of Formula (2'). 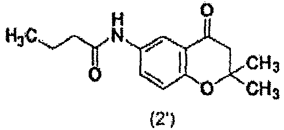
(VII) A compound of Formula (3'). 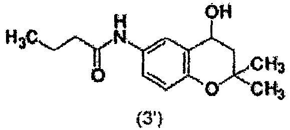
(VIII) A compound of Formula (4').

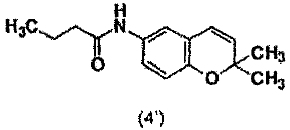

### Effects of the Invention

The present invention can provide a method for producing a heterocyclic compound that is suitable for production in an industrial scale since the method is performed for a short time at a low cost as compared with the conventional production method.

### MODES FOR CARRYING OUT THE INVENTION

Hereinafter, the present invention will be described. However, the present invention is not limited to the following embodiments, and various modifications can be made within a scope of the present invention described in claims.

In the present specification, "n-" means normal, "i-" means iso, "s-" means secondary, and "t-" means tertiary.

In the present specification, a C₁₋₆ alkyl group means an alkyl group having a carbon atom number of 1 to 6. The alkyl group may be linear or branched. Examples thereof include methyl group, ethyl group, n-propyl group, i-propyl group, n-butyl group, i-butyl group, s-butyl group, t-butyl group, n-pentyl group, and n-hexyl group.

The C₁₋₆ alkyl group in R¹ is preferably a C₂₋₄ alkyl group (alkyl group having a carbon atom number of 2 to 4), more preferably ethyl group, n-propyl group, n-butyl group, or i-propyl group, and further preferably n-propyl group.

The following scheme shows the outlines of each production process in a production method of the present invention. An example in which in the following scheme, compounds (1') to (4') of Formulae (1) to (4), respectively, wherein R¹ is a n-propyl group are described as specific examples of compounds (1) to (4), respectively, and maleic acid is used as a salt of a compound (6) will be described.

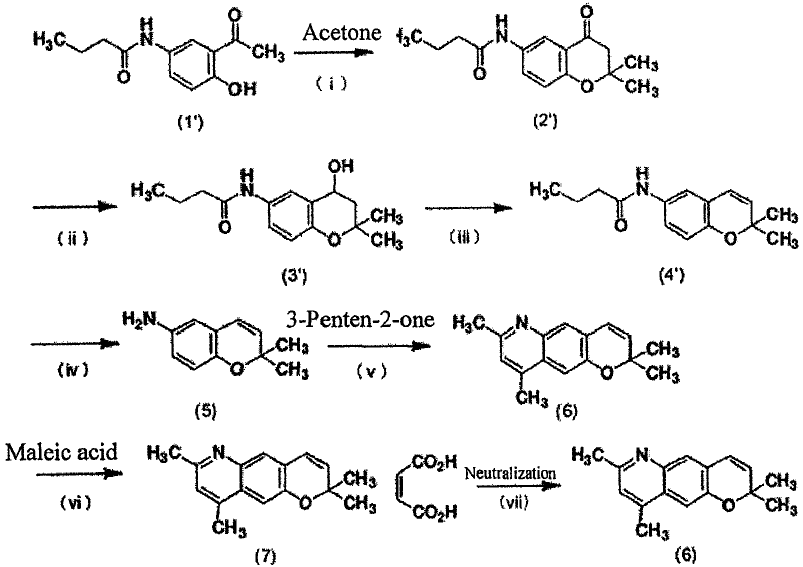

Hereinafter in the present specification, as necessary, a step (i) is referred to as "chromanone cyclization step," a step (ii) is referred to as "reduction step," a step (iii) is referred to as "dehydration step," a step (iv) is referred to as "hydrolysis step," a step (v) is referred to as "quinoline cyclization step," a step (vi) is referred to as "salt forming step," and a step (vii) is referred to as "neutralization step." Further, a reaction at the step (i) is referred to as "chromanone cyclization reaction," a reaction at the step (ii) is referred to as "reduction reaction," a reaction at the step (iii) is referred to as "dehydration reaction," a reaction at the step (iv) is referred to as "hydrolysis reaction," a reaction at the step (v) is referred to as "quinoline cyclization reaction," a reaction at the step (vi) is referred to as "salt forming reaction," and a reaction at the step (vii) is referred to as "neutralization reaction."

Hereinafter, the respective steps will be described in order. The order of adding a solvent and a reagent, and operation order shown below are not limited, and the orders may be modified and carried out.

### (i) Chromanone Cyclization Step

A chromanone cyclization step of reacting the compound (1) with acetone to obtain a chromanone ring derivative (compound (2)) will be described. It is preferable that the compound (1) be first dissolved in a solvent and reacted with acetone.

The solvent usable in the chromanone cyclization step is not limited as long as effects of the object of the present invention can be achieved. In terms of effectively carrying out the reaction, an alcohol solvent (e.g., methanol, ethanol, n-propanol, and i-propanol) is preferably used. Among them, methanol, ethanol, or n-propanol is more preferably used.

The amount of the solvent to be used is not particularly limited, and is 0.1 mass times to 100 mass times, preferably 0.5 mass times to 50 mass times, and more preferably 1 mass time to 20 mass times, relative to the amount of the compound (1).

The amount of acetone to be reacted with the compound (1) is not particularly limited, and is 0.1 molar equivalents to 20 molar equivalents, preferably 0.5 molar equivalents to 10 molar equivalents, and more preferably 1 molar equivalent to 5 molar equivalents, relative to the amount of the compound (1).

It is preferable that this step be carried out in the presence of a base in terms of enhancing the reactivity. The base is preferably an organic base, more preferably a 5- or 6-membered nitrogen-containing cyclic organic base, and further preferably pyrrolidine.

The amount of the base to be used is not particularly limited, and is 0.1 molar equivalents to 10 molar equivalents, preferably 0.5 molar equivalents to 5 molar equivalents, and more preferably 1 molar equivalent to 3 molar equivalents, relative to the amount of the compound (1).

The reaction temperature in the chromanone cyclization step is not particularly limited, and is preferably any temperature within a range of 0°C to the boiling point of the solvent used. In terms of effectively carrying out the reaction, it is preferable that the reaction temperature be any temperature within a range of 50°C to the boiling point of the solvent used.

The reaction time in the chromanone cyclization step is not particularly limited as long as it is sufficient to consume a reactant, and is preferably 10 minutes to 24 hours, and more preferably 30 minutes to 6 hours.

After the reaction, the obtained compound (2) can be isolated or purified by an ordinary method. For example, the isolation or purification can be carried out by a known method such as extraction by the solvent, silica gel column chromatography, high-performance liquid chromatography, and crystallization. Alternatively, after the reaction, a solution of the obtained compound (2) can be used as it is in the next step.

### (ii) Reduction Step

Next, the reduction step in which a carbonyl group of the chromanone derivative (compound (2)) obtained in the previous step is reduced to obtain an alcohol derivative (compound (3)) will be described. It is preferable that the compound (2) be first dissolved in a solvent, followed by carrying out a reduction reaction.

The solvent usable in the reduction step is not limited as long as the effects of the object of the present invention can be achieved. In terms of effectively carrying out the reaction, an alcohol solvent (e.g., methanol, ethanol, n-propanol, and i-propanol), tetrahydrofuran, or a mixed solvent thereof is preferably used. Among them, methanol, ethanol, a mixed solvent of methanol and tetrahydrofuran, or a mixed solvent of ethanol and tetrahydrofuran is more preferably used, and a mixed solvent of methanol and tetrahydrofuran is particularly preferably used.

The amount of the solvent to be used is not particularly limited, and is 0.1 mass times to 100 mass times, preferably 0.5 mass times to 20 mass times, and more preferably 1 mass time to 10 mass times, relative to the amount of the compound (2).

A reductant used in this step is not particularly limited as long as it is a reductant of promoting a desired reaction. The reductant is preferably an aluminum metal hydride salt or a metal borohydride salt, more preferably a metal borohydride salt, and further preferably sodium borohydride.

The amount of the reductant to be used is not particularly limited, and is 0.1 molar equivalents to 5 molar equivalents, preferably 0.5 molar equivalents to 3 molar equivalents, and more preferably 0.5 molar equivalents to 1.5 molar equivalents, relative to the amount of the compound (2).

The reaction temperature in the reduction step is not particularly limited, and is preferably any temperature within a range of 0°C to the boiling point of the solvent used, and more preferably any temperature within a range of 1°C to 60°C.

The reaction time in the reduction step is not particularly limited as long as it is sufficient to consume a reactant, and is preferably 10 minutes to 24 hours, and more preferably 30 minutes to 6 hours.

After the reaction, the obtained compound (3) can be isolated or purified by an ordinary method. For example, the compound (3) can be isolated or purified by a known method such as extraction by the solvent, silica gel column chromatography, and high-performance liquid chromatography. The reaction solution is cooled, an acid and a base are added to make the property of the solution neutral and precipitate a crystal of the compound (3). After then, the compound can be isolated or purified. Alternatively, after the reaction, a solution of the obtained compound (3) can be used as it is in the next step.

In a method of the isolation or purification using an acid and a base, the temperature of the reaction solution during the isolation or purification is not particularly limited, and is preferably any temperature within a range of -20°C to the boiling point of the solvent used, more preferably any temperature within a range of -10°C to 40°C, and further preferably any temperature within a range of -5°C to 20°C.

In the method using an acid and a base, the acid used is not particularly limited, and hydrochloric acid is preferably used. The base used is not particularly limited, and sodium hydrogen carbonate is preferably used. The acid and base can be appropriately used in amounts required to make the property of the solution neutral.

The stirring time after addition of the base during the isolation or purification is not particularly limited. From the viewpoint of production efficiency, it is preferable that the reaction solution be stirred for any time within 24 hours immediately after addition of the base, and more preferably for 1 hour to 10 hours.

A crystal of the isolated compound (3) may be dried, or used without drying (with moist) as it is in the next step.

### (iii) Dehydration Step

Next, the dehydration step in which the alcohol derivative (compound (3)) obtained in the previous step is dehydrated to obtain a chromene ring derivative (compound (4)) will be described. This step can be also carried out simultaneously with the hydrolysis step (iv) described below.

In this step, the compound (3) obtained after the reduction step may be isolated and used, or the reaction solution may be used as it is.

When the isolated or purified compound (3) is used, it is preferable that the compound (3) be dissolved in a solvent, followed by carrying out a dehydration reaction.

The solvent used in dissolution of the compound (3) is not limited as long as the effects of the object of the present invention can be achieved. In terms of effectively carrying out the reaction, an alcohol solvent (e.g., methanol, ethanol, n-propanol, and i-propanol), or an aromatic hydrocarbon solvent (e.g., benzene, toluene, and xylene) is preferably used. Among them, methanol, ethanol, n-propanol, or toluene is more preferably used, and toluene is particularly preferably used. When the dehydration step is carried out simultaneously with the hydrolysis step (iv) described below, an alcohol solvent (e.g., methanol, ethanol, n-propanol, and i-propanol) is preferably used as the solvent, in terms of effectively carrying out the reaction. Among them, methanol, ethanol, or n-propanol is preferably used, and ethanol is particularly preferably used.

The amount of the solvent to be used is not particularly limited, and is 0.1 mass times to 100 mass times, preferably 0.5 mass times to 50 mass times, and more preferably 1 mass time to 30 mass times, relative to the amount of the compound (3).

It is preferable that this step be carried out in the presence of an acid in terms of enhancing the reactivity. The acid usable when the dehydration step is carried out independently is not limited as long as the effects of the object of the present invention can be achieved. In terms of effectively carrying out the reaction, it is preferable the acid be hydrochloric acid or methanesulfonic acid. When the dehydration step is carried out simultaneously with the hydrolysis step (iv) described below, hydrochloric acid is preferably used as the acid.

The amount of the acid to be used is not particularly limited, and is 0.01 molar equivalents to 100 molar equivalents, preferably 0.03 molar equivalents to 20 molar equivalents, and more preferably 0.05 molar equivalents to 10 molar equivalents, relative to the amount of the compound (3).

The reaction temperature in this step is not particularly limited, and is preferably any temperature within a range of 0°C to the boiling point of the solvent used, more preferably any temperature within a range of 1°C to the boiling point of the solvent, and further preferably any temperature within a range of 60°C to the boiling point of the solvent.

The reaction time in this step is not particularly limited as long as it is sufficient to consume a reactant, and is preferably 10 minutes to 72 hours, and more preferably 0.5 hours to 48 hours.

After the reaction, the obtained compound (4) can be isolated or purified by an ordinary method. For example, the isolation or purification can be carried out by a known method such as extraction by the solvent, silica gel column chromatography, high-performance liquid chromatography, and crystallization. Alternatively, after the reaction, a solution of the obtained compound (4) can be used as it is in the next step.

### (iv) Hydrolysis Step

Next, the hydrolysis step in which the chromene ring derivative (compound (4)) obtained in the previous step is hydrolyzed to obtain 2,2-dimethyl-2H-chromen-6-amine (compound (5)) will be described. It is preferable that the compound (4) obtained in the dehydration step be dissolved in a solvent, followed by carrying out a hydrolysis reaction.

The solvent used in dissolution of the compound (4) is not limited as long as the effects of the object of the present invention can be achieved. In terms of effectively carrying out the reaction, it is preferable that the solvent be an alcohol solvent (e.g., methanol, ethanol, n-propanol, and i-propanol). Among them, methanol, ethanol, or n-propanol is preferably used.

The amount of the solvent to be used is not particularly limited, and is 0.1 mass times to 100 mass times, preferably 0.5 mass times to 50 mass times, and more preferably 1 mass time to 30 mass times, relative to the amount of the compound (4).

It is preferable that this step be carried out in the presence of an acid in terms of enhancing the reactivity. The acid usable in the hydrolysis step alone is not limited as long as the effects of the object of the present invention can be achieved. In terms of effectively carrying out the reaction, it is preferable that the acid be hydrochloric acid. Hydrochloric acid is also suitable as the acid in the dehydration step (iii). Therefore, when the dehydration step and the hydrolysis step are carried out simultaneously, it is suitable that hydrochloric acid be used as the acid.

The amount of the acid to be used is not particularly limited, and is 0.1 molar equivalents to 100 molar equivalents, preferably 0.5 molar equivalents to 50 molar equivalents, and more preferably 1 molar equivalent to 20 molar equivalents, relative to the amount of the compound (4).

The reaction temperature in this step is not particularly limited, and is preferably any temperature within a range of 0°C to the boiling point of the solvent used, more preferably any temperature within a range of 1 °C to the boiling point of the solvent, and further preferably any temperature within a range of 60°C to the boiling point of the solvent.

The reaction time in this step is not particularly limited as long as it is sufficient to consume a reactant, and is preferably 10 minutes to 48 hours, and more preferably 0.5 hours to 24 hours.

The obtained compound (5) can be isolated or purified by an ordinary method. For example, the isolation or purification can be carried out by a known method such as extraction by the solvent, silica gel column chromatography, high-performance liquid chromatography, and crystallization. Alternatively, after the reaction, a solution of the obtained compound (5) can be used as it is in the next step.

### (v) Quinoline Cyclization Step

Next, the quinoline cyclization step in which the 2,2-dimethyl-2H-chromen-6-amine (compound (5)) obtained in the previous step is reacted with 3-penten-2-one for cyclization, obtaining 2,2,7,9-tetramethyl-2H-pyrano[2,3-g]quinoline (compound (6)) will be described. It is preferable that the compound (5) obtained in the hydrolysis step (iv) be first dissolved in a solvent, followed by carrying out a quinoline cyclization reaction.

The solvent usable in the quinoline cyclization step is not limited as long as the effects of the object of the present invention can be achieved. In terms of effectively carrying out the reaction, an alcohol solvent (e.g., methanol, ethanol, n-propanol, and i-propanol) is preferably used. Among them, ethanol or n-propanol is preferably used.

The amount of the solvent to be used is not particularly limited, and is 0.1 mass times to 100 mass times, preferably 0.5 mass times to 50 mass times, and more preferably 1 mass time to 20 mass times, relative to the amount of the compound (5).

The amount of 3-penten-2-one to be reacted with the compound (5) is not particularly limited, and is 0.1 molar equivalents to 10 molar equivalents, preferably 1 molar equivalent to 5 molar equivalents, and more preferably 1 molar equivalent to 3 molar equivalents, relative to the amount of the compound (5).

In this step, the reaction can be carried out in the presence or absence of an acid. In terms of enhancing the reactivity, it is preferable that the reaction be carried out in the presence of an acid. The acid usable in the quinoline cyclization step is not limited as long as the effects of the object of the present invention can be achieved, and hydrochloric acid is preferred.

The amount of the acid to be used is not particularly limited, and is 0.1 molar equivalents to 20 molar equivalents, preferably 0.5 molar equivalents to 10 molar equivalents, and more preferably 1 molar equivalent to 7 molar equivalents, relative to the amount of the compound (5).

In this step, it is preferable that the reaction be carried out in the presence of an oxidant in terms of enhancing the reactivity. The oxidant usable in the quinoline cyclization step is not limited as long as the effects of the object of the present invention can be achieved. It is preferable that the oxidant be a copper salt, an iron salt, or quinones, and more preferably iron (II) chloride, iron (III) chloride, or anthraquinone. In terms of effectively carrying out the reaction, it is further preferably that the oxidant be iron (III) chloride.

The amount of the oxidant to be used is not particularly limited, and is 0.1 molar equivalents to 20 molar equivalents, preferably 1 molar equivalent to 10 molar equivalents, and more preferably 1 molar equivalent to 5 molar equivalents, relative to the amount of the compound (5).

The reaction temperature in the quinoline cyclization step is not particularly limited, and is preferably any temperature within a range of 0°C to the boiling point of the solvent used, more preferably any temperature within a range of 1°C to the boiling point of the solvent, and further preferably any temperature within a range of 60°C to the boiling point of the solvent.

The reaction time in the quinoline cyclization step is not particularly limited as long as it is sufficient to consume a reactant, and is preferably 10 minutes to 24 hours, and more preferably 1 hour to 15 hours.

The obtained compound (6) can be isolated or purified by an ordinary method. For example, the compound (6) can be isolated and purified by a known method such as extraction by the solvent, silica gel column chromatography, high-performance liquid chromatography, and crystallization. Alternatively, after the reaction, a solution of the obtained compound (6) can be used as it is in the next step.

### (vi) Salt Forming Step

Next, the salt forming step in which 2,2,7,9-tetramethyl-2H-pyrano[2,3-g]quinoline (compound (6)) obtained in the previous step is reacted with an acid to produce a crystal of salt of 2,2,7,9-tetramethyl-2H-pyrano[2,3-g]quinoline will be described. The produced crystal of salt is neutralized to obtain 2,2,7,9-tetramethyl-2H-pyrano[2,3-g]quinoline (compound (6)) having higher purity.

It is preferable that the compound (6) be first dissolved in a solvent, followed by carrying out formation of salt.

The solvent usable in the salt forming step is not limited as long as the effects of the object of the present invention can be achieved. An aromatic hydrocarbon solvent (e.g., benzene, toluene, and xylene) is preferably used in terms of effectively carrying out a post-treatment. Among them, toluene is more preferably used.

The amount of the solvent to be used is not particularly limited, and is 0.1 mass times to 20 mass times, preferably 1 mass time to 10 mass times, and more preferably 1 mass time to 5 mass times, relative to the amount of the compound (6).

The acid usable in the salt forming step is not limited as long as the effects of the object of the present invention can be achieved, and maleic acid is preferably used.

The amount of the acid to be used is not particularly limited, and is 0.1 molar equivalents to 10 molar equivalents, preferably 0.5 molar equivalents to 5 molar equivalents, and more preferably 0.5 molar equivalents to 3 molar equivalents, relative to the amount of the compound (6).

The acid used in the salt forming step is used in a solution form. A solvent used for the dissolution is not limited as long as the effects of the object of the present invention can be achieved. An alcohol solvent (e.g., methanol, ethanol, n-propanol, and i-propanol) is preferably used in terms of effectively forming a salt. Among them, methanol, ethanol, or n-propanol is preferably used, and ethanol is particularly preferably used.

The amount of the solvent of dissolving the acid to be used is not particularly limited, and is 0.1 mass times to 100 mass times, preferably 0.5 mass times to 20 mass times, and more preferably 1 mass time to 10 mass times, relative to the amount of the compound (6).

The temperature during the reaction of the compound (6) with the acid is not particularly limited, and is preferably any temperature within a range of 0°C to the boiling point of the solvent used, more preferably within a range of 1 °C to the boiling point of the solvent, and further preferably within a range of 1°C to 60°C.

The reaction time in the salt forming step is not particularly limited as long as it is sufficient to consume a reactant, and is preferably any time within 12 hours immediately after addition of the acid, and more preferably 1 minute to 3 hours.

Subsequently, the reaction solution is cooled. The cooling temperature is not particularly limited, and is preferably within a range of -20°C to 10°C, and more preferably within a range of -15°C to 5°C.

The cooling time of the reaction solution is not particularly limited, and is preferably any time of 0.5 hours to 24 hours.

After the cooling, the produced crystal of salt of 2,2,7,9-tetramethyl-2H-pyrano[2,3-g]quinoline (in a case of using maleic acid as the acid, crystal of a compound (7)) is filtered and dried to obtain the crystal of the purified salt.

### (vii) Neutralization Step

Next, the neutralization step in which the crystal of salt of 2,2,7,9-tetramethyl-2H-pyrano[2,3-g]quinoline (compound (7)) obtained in the previous step is reacted with a base to produce 2,2,7,9-tetramethyl-2H-pyrano[2,3-g]quinoline (compound (6)) will be described.

It is preferable that the compound (7) be first mixed in a solvent, followed by carrying out neutralization.

The solvent usable in the neutralization step is not limited as long as the effects of the object of the present invention can be achieved. An aromatic hydrocarbon solvent (e.g., benzene, toluene, and xylene) is preferably used in terms of effectively carrying out a post-treatment. Among them, toluene is more preferably used.

The amount of the solvent to be used is not particularly limited, and is 0.1 mass times to 50 mass times, preferably 1 mass time to 20 mass times, and more preferably 1 mass time to 10 mass times, relative to the amount of the compound (7).

The base usable in the neutralization step is not limited as long as the effects of the object of the present invention can be achieved. An inorganic base is preferably used in terms of effectively carrying out the post-treatment. Among them, sodium hydroxide, potassium hydroxide, sodium carbonate, potassium carbonate, sodium hydrogen carbonate, or potassium hydrogen carbonate is preferably used, and sodium hydrogen carbonate is particularly preferably used.

The amount of the base to be used is not particularly limited, and is 0.1 molar equivalents to 20 molar equivalents, preferably 1 molar equivalent to 10 molar equivalents, and more preferably 2 molar equivalents to 5 molar equivalents, relative to the amount of the compound (7).

The base used in the neutralization step is used in a solution form. A solvent used for the dissolution is not limited as long as the effects of the object of the present invention can be achieved. An alcohol solvent (e.g., methanol, ethanol, n-propanol, and i-propanol) or water is preferably used in terms of effectively carrying out neutralization. Among them, water is more preferably used.

The amount of the solvent of dissolving the base to be used is not particularly limited, and is 0.1 mass times to 100 mass times, preferably 0.5 mass times to 50 mass times, and more preferably 1 mass time to 20 mass times, relative to the amount of the compound (7).

The temperature during the reaction of the compound (7) with the base is not particularly limited, and is preferably any temperature within a range of 0°C to the boiling point of the solvent used, more preferably within a range of 10°C to 60°C, and further preferably within a range of 20°C to 40°C.

The reaction time in the neutralization step is not particularly limited as long as it is sufficient to consume a reactant, and is preferably any time within 12 hours immediately after addition of the base, and more preferably 1 minute to 3 hours.

The obtained compound (6) can be isolated or purified by an ordinary method. For example, the compound (6) can be isolated or purified by a known method such as extraction by the solvent, and silica gel column chromatography. The purity of the compound (6) obtained at the neutralization step can be made higher than that of 2,2,7,9-tetramethyl-2H-pyrano[2,3-g]quinoline (compound (6)) obtained in the step (v).

### Examples

Hereinafter, the present invention will be described specifically with reference to Examples. However, the scope of the present invention is not limited to these examples.

In the present specification, filtration through a pad of silica gel is a treatment process in which a reaction solution (or suspension) is passed through a filter including a layer of silica gel to remove a solid material and a component absorbed on the silica gel from the reaction solution.

The structure of each of the compounds obtained in Examples was identified by appropriate combination of NMR analysis, mass spectroscopy, and the like, the purity of each compound was calculated by HPLC, and the melting point of each compound was measured.

In Examples, NMR means nuclear magnetic resonance, HPLC means high-performance liquid chromatography, V/V means volume vs volume, ESI means electrospray ionization, "ESI+" means ESI positive ion mode, and "ESI-" means ESI negative ion mode.

NMR analysis was carried out using ECP300 manufactured by JEOL Ltd., mass spectroscopy was carried out using MICROMASS ZQ manufactured by WATERS Corporation, and the melting point was measured using B-545 manufactured by SIBATA SCIENTIFIC TECHNOLOGY LTD.

¹H-NMR data described is a chemical shift δ (unit: ppm) (division pattern, integrated value) of signal using tetramethylsilane as an internal standard. "s" means singlet, "d" means doublet, "t" means triplet, "q" means quartet, "dd" means doublet of doublet, "qt" means quartet of triplet, "m" means multiplet, "br" means broad, "J" means coupling constant, and "CDCl₃" means deuterated chloroform.

HPLC analysis was carried out using LC-20A manufactured by Shimadzu Corporation under the following conditions.

The purity (in a case of impurity, content ratio) of each compound by HPLC analysis is expressed by an area percentage method by which a ratio of the target peak area in the total peak area is expressed in percentage.

### [Examples 1 to 5]

L-column ODS (manufactured by Chemicals Evaluation and Research Institute, Japan, 4.6 mm in diameter × 250 mm in length, particle diameter: 5 µm)
Eluent: acetonitrile/aqueous solution of 0.01 M ammonium acetate =22/78 to 45/55 (0 to 12 minutes), 45/55 (12 to 22 minutes), 45/55 to 95/5 (22 to 45 minutes), and 95/5 (45 to 65 minutes), V/V
Flow rate: 1.0 mL/min
Column temperature: 40°C
Wavelength of ultraviolet-visible spectroscopy: 238 nm

### [Examples 6 and 7]

L-column ODS (manufactured by Chemicals Evaluation and Research Institute, Japan, 4.6 mm in diameter × 250 mm in length, particle diameter: 5 µm)
Eluate: 400 mL of acetonitrile and 600 mL of 0.01 M acetic acid buffer solution (pH: 3.8) were mixed and 0.29 g of sodium dodecylsulfate was dissolved in the mixture. As the 0.01 M acetic acid buffer solution, a mixture of 800 mL of aqueous solution of 0.01 M acetic acid and 100 mL of solution of 0.01 M sodium acetate was used.
Flow rate: 1.0 mL/min
Column temperature: 40°C
Wavelength of ultraviolet-visible spectroscopy: 254 nm

### Example 1

### Method for Producing Compound (2'): N-(2,2-dimethyl-4-oxochroman-6-yl)butylamide

N-(3-acetyl-4-hydroxyphenyl)butylamide (compound (1')) (2.01 g, 9.08 mmol), n-propanol (20.0 g), acetone (1.60 g, 27.6 mmol), and pyrrolidine (1.30 g, 18.3 mmol) were mixed, heated at a temperature range of 97°C to 100°C, and stirred for 2 hours. After then, hydrochloric acid and ethyl acetate were added, and the mixture was subjected to phase separation to obtain an organic phase. The obtained organic phase was subjected to phase separation with water. To the resulting aqueous phase, ethyl acetate was added, and the mixture was subjected to phase separation to obtain an organic phase. The obtained organic phase was combined with the organic phase obtained earlier, concentrated, filtered through a pad of silica gel, washed with a solution of ethyl acetate and hexane at a ratio of 1 : 1, and concentrated again, to obtain 2.40 g of compound (2') as a crude product.

### Compound (2')

MASS (ESI⁺) m/z; 262 (M+1)⁺

¹H-NMR (CDCl₃, TMS):
δ (ppm): 1.01 (3H, t, J=7.2 Hz), 1.45 (6H, s), 1.76 (2H, qt, J=7.5, 7.2 Hz), 2.33 (2H, t, J=7.5 Hz), 2.71 (2H, s), 6.91 (1H, d, J=8.9 Hz), 7.35 (1H, br-s), 7.67 (1H, d, J=2.7 Hz), 7.94 (1H, dd, J=8.9, 2.7 Hz)

### Example 2

### Method for Producing Compound (3'): N-(4-hydroxy-2,2-dimethylchroman-6-yl)butylamide

The compound (2') (1.20 g, 4.59 mmol) obtained in Example 1, tetrahydrofuran (5.26 g), and methanol (0.71 g) were mixed, and cooled to 2°C. Sodium borohydride (0.195 g, 5.15 mmol) was added, and the mixture was stirred at a temperature range of 11°C to 17°C for 1 hour. After then, ethyl acetate (20 mL) and hydrochloric acid (20 mL) were added, and the mixture was subjected to phase separation to obtain an organic phase. To the resulting aqueous phase, ethyl acetate was added, and the mixture was further subjected to phase separation to obtain an organic phase. The organic phase was combined with the organic phase obtained earlier, and was subjected to phase separation with water to obtain an organic phase. The finally obtained organic phase was concentrated to obtain 1.40 g of compound (3') as a crude product.

### Compound (3')

MASS (ESI⁺) m/z; 264 (M+1)⁺

¹H-NMR (CDCl₃, TMS):
δ (ppm): 0.89 (3H, t, J=7.4 Hz), 1.20 (3H, s), 1.33 (3H, s), 1.58 (2H, qt, J=7.4, 7.2 Hz), 1.71 to 1.63 (1H, m), 2.02 (1H, dd, J=13.2, 6.1 Hz), 2.21 (2H, t, J=7.2 Hz), 4.60 (1H, t, J=9.1 Hz), 5.28 (1H, br-s), 6.59 (1H, d, J=8.4 Hz), 7.29 (1H, dd, J=8.8,2.5 Hz), 7.63 (1H, d, J=2.5 Hz), 9.61 (1H, s)

### Example 3

### Method for Producing Compound (4'): N-(2,2-dimethyl-2H-chromen-6-yl)butylamide

The compound (3') (0.95 g, 3.61 mmol) obtained in Example 2, toluene (20.0 g), and methanesulfonic acid (0.10 g, 0.30 mmol) were mixed, and heated to 110°C. The mixture was stirred for 1 hour. After then, water (80 mL) and ethyl acetate (80 mL) were added, and the mixture was subjected to phase separation to obtain an organic phase. The resulting aqueous phase was further subjected to phase separation with ethyl acetate to obtain an organic phase. The obtained organic phase was combined with the organic phase obtained earlier, concentrated, and purified by column chromatography, to obtain 0.75 g of compound (4').

### Compound (4')

MASS (ESI⁺) m/z; 246 (M+1)⁺

¹H-NMR (CDCl₃, TMS):
δ (ppm): 1.00 (3H, t, J=7.4 Hz), 1.41 (6H, s), 1.75 (2H, qt, J=7.4, 7.2 Hz), 2.30 (2H, t, J=7.2 Hz), 5.62 (1H, d, J=9.9 Hz), 6.28 (1H, d, J=9.6 Hz), 6.71 (1H, d, J=8.5 Hz), 7.06 (1H, dd, J=8.5, 2.2 Hz), 7.07 (1H, br-s), 7.30 (1H, d, J=2.2 Hz)

### Example 4

### Method for Producing Compound (5): 2,2-dimethyl-2H-chromen-6-amine

The compound (4') (0.22 g, 0.88 mmol) obtained in Example 3, ethanol (5.00 g), and concentrated hydrochloric acid (1.02 g, 9.8 mmol) were mixed, and heated to 80°C. The mixture was stirred for 13 hours and 30 minutes. After then, ethyl acetate, water, and an aqueous sodium hydroxide solution were added, and the mixture was subjected to phase separation to obtain an organic phase. The resulting aqueous phase was further subjected to phase separation with ethyl acetate twice, and each organic phase was collected. All the obtained organic phases were combined and concentrated to obtain 0.19 g of compound (5) as a crude product.

### Compound (5)

MASS (ESI⁺) m/z; 176 (M+1)⁺

¹H-NMR (CDCl₃, TMS):
δ (ppm): 1.39 (6H, s), 3.35 (2H, br-s), 5.60 (1H, d, J=9.9 Hz), 6.23 (1H, d, J=9.9 Hz), 6.37 (1H, d, J=2.8 Hz), 6.47 (1H, dd, J=8.5, 2.8 Hz), 6.61 (1H, d, J=8.3 Hz)

### Example 5

### Method for Producing compound (6): 2,2,7,9-tetramethyl-2H-pyrano[2,3-g]quinoline

The compound (5) (0.18 g, 1.00 mmol) obtained in Example 5, n-propanol (1.80 g), concentrated hydrochloric acid (0.38 g, 3.65 mmol), and iron (III) chloride (0.50 g, 3.08 mmol) were mixed, and the mixture was heated to 76°C. Further, 3-penten-2-one (0.19 g, 1.83 mmol) was added, and the mixture was stirred at a temperature range of 80°C to 95°C for 3 hours. After then, an aqueous sodium carbonate solution and ethyl acetate were added and the mixture was subjected to phase separation to obtain an organic phase. The resulting aqueous phase was further subjected to phase separation with ethyl acetate to obtain an organic phase. All the obtained organic phases were combined, concentrated, and purified by column chromatography, to obtain 0.13 g of compound (6).

### Compound (6)

MASS: 240 (M+1)

¹H-NMR (CDCl₃, TMS):
δ (ppm): 1.49 (6H, s), 2.54 (3H, s), 2.62 (3H, s), 5.86 (1H, d, J=9.9 Hz), 6.56 (1H, d, J=9.9 Hz), 7.01 (1H, s), 7.20 (1H, s), 7.60 (1H, s)
Melting Point: 64°C

### Example 6

### Method for Producing Compound (3'): N-(4-hydroxy-2,2-dimethylchroman-6-yl)butylamide

N-(3-acetyl-4-hydroxyphenyl)butylamide (compound (1')) (160 g, 0.72 mol), methanol (480 g), and pyrrolidine (77.3 g, 1.08 mol) were mixed, and heated to 43°C. Acetone (84.7 g, 1.45 mol) was added, and the mixture was stirred at a temperature range of 44°C to 45°C for 3 hours.

After the stirring, the mixture was cooled, and tetrahydrofuran (320 g) was added. The temperature of the reaction solution was cooled to 33°C, and then sodium borohydride (27.4 g, 0.72 mol) was added in ten divided portions. The mixture was stirred at a temperature range of 36°C to 37°C for 3 hours. The mixture was then cooled to 6°C, 10% hydrochloric acid (634 g) was added, and the mixture was stirred for 1 hour. An aqueous solution (365 g) of 5% sodium hydrogen carbonate was then added, the mixture was stirred for 2 hours, and the precipitated crystal was filtered. After the filtration, the resulting crystal was washed with a mixed liquid of ethanol (161 g) and water (161 g), and again washed with the mixed liquid in the same amount. 313 g of wet crystal of the compound (3') (purity: 99.97%) was obtained as a white crystal. A part of the wet crystal was collected and dried, and the calculated dry weight was 165 g.

### Example 7

### Method for Producing compound (7): 2,2,7,9-tetramethyl-2H-pyrano[2,3-g]quinoline malate

The compound (3') (140 g, 0.53 mol; (which is the amount of dried crystal; 177 g in a case of wet crystal of the compound (3'))) obtained in Example 6 and ethanol (662 g) were mixed, and the mixture was heated to 77°C. Concentrated hydrochloric acid (277 g, 2.66 mol) was added, and the mixture was stirred at a temperature range of 80°C to 81°C for 13 hours, and then cooled to 30°C.

To the obtained reaction solution, iron (III) chloride (259 g, 1.60 mol) was added, and the mixture was heated to 82°C. Further, 3-penten-2-one (76.0 g, 0.90 mol) was added, and the mixture was stirred at a temperature range of 84°C to 85°C for 4 hours. The mixture was then cooled, toluene (700 g) and water (420 g) were added, and the mixture was stirred and then subjected to phase separation to obtain an organic phase. To the obtained organic phase, an aqueous solution (1,343 g) of 17% potassium carbonate was added, and the mixture was stirred and further subjected to phase separation to obtain an organic phase. To the resulting aqueous phase, toluene (700 g) was added, and the mixture was stirred and then subjected to phase separation to obtain an organic phase. All the organic phases thus obtained were combined, and water (700 g) was added. The mixture was stirred and then subjected to phase separation to obtain an organic phase. To the obtained organic phase, activated carbon (7.00 g) was added, and the mixture was stirred for 1 hour, and filtered through Celite as a filter aid. Further, the Celite was washed with toluene (140 g) and the filtrate was obtained. The filtrate after the filtration was concentrated to obtain 560 g of solution of the compound (6) in toluene (purity: 85.07%). (Here, the purity was calculated excluding the peak of toluene.)

Subsequently, to maleic acid (49.4 g, 0.43 mol), ethanol (280 g) was added and the mixture was dissolved. The resulting mixture was added to the solution of the compound (6) in toluene of 50°C, and the mixture was cooled from 50°C to 2°C, and stirred at 2°C for 1 hour. After the stirring, the precipitated crystal was filtered. After the filtration, the resulting crystal was washed with a mixed liquid of toluene (98 g) and ethanol (42 g), and again washed with the mixed liquid in the same amount. The washed crystal was dried to obtain 111 g of compound (7) (purity: 96.81%) as a brown crystal. (Here, the purity was calculated excluding the peak of maleic acid.)

### Compound (7)

MASS (ESI+) m/z; 240 (M+1)⁺

¹H-NMR (CDCl₃, TMS):
δ (ppm): 1.46 (6H, s), 2.63 (3H, s), 2.65 (3H, s), 6.16 (2H, s), 6.17 (1H, d, J=9.6 Hz), 6.75 (1H, d, J=9.9 Hz), 7.35 (1H, s), 7.39 (1H, s), 7.67 (1H, s) Melting Point: 188°C

### Example 8

### Method for Producing compound (6): 2,2,7,9-tetramethyl-2H-pyrano[2,3-g]quinoline

The compound (7) (5.01 g, 14.1 mmol, purity: 96.81% (except for the peak of maleic acid)) obtained in Example 7 and toluene (25.10 g) were mixed. A mixed solution of sodium hydrogen carbonate (3.00 g, 35.2 mmol) and water (50.00 g) was added at 25°C, and the mixture was stirred at 25°C for 1 hour and subjected to phase separation to obtain an organic phase.

To the obtained organic phase, water (25.02 g) was added, and the mixture was stirred and then subjected to phase separation to obtain an organic phase. To the obtained organic phase, anhydrous magnesium sulfate was added. The resulting mixture was dried and filtered. After the filtration, a filtrate was concentrated to obtain 3.20 g of the compound (6) (purity: 96.26%).

### Compound (6)

MASS: 240(M+1)

¹H-NMR (CDCl₃, TMS):
δ (ppm): 1.49 (6H, s), 2.54 (3H, s), 2.62 (3H, s), 5.86 (1H, d, J=9.9 Hz), 6.56 (1H, d, J=9.9 Hz), 7.01 (1H, s), 7.20 (1H, s), 7.60 (1H, s)
Melting Point: 64°C

### INDUSTRIAL APPLICABILITY

The present invention provides the method for producing a heterocyclic compound.

## Claims

1. A method for producing 2,2-dimethyl-2H-chromen-6-amine comprising the following steps (a) to (d) (in the following formulae (1) to (4), R¹ is a C₁₋₆ alkyl group):
(a) reacting a compound of Formula (1): 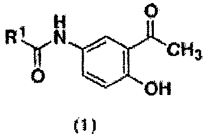 with acetone to obtain a chromanone ring derivative of Formula (2): 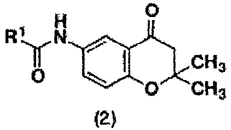
(b) reducing the obtained chromanone ring derivative to obtain an alcohol derivative of Formula (3): 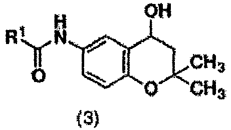
(c) dehydrating the obtained alcohol derivative to obtain a chromene ring derivative of Formula (4): 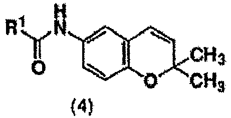 and
(d) hydrolyzing the obtained chromene ring derivative to obtain 2,2-dimethyl-2H-chromen-6-amine of Formula (5).

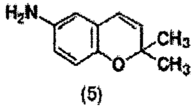

2. The method according to claim 1, wherein R¹ is a n-propyl group.

3. A method for producing 2,2,7,9-tetramethyl-2H-pyrano[2,3-g]quinoline comprising the following steps (e) and (f):
(e) reacting 2,2-dimethyl-2H-chromen-6-amine of Formula (5): 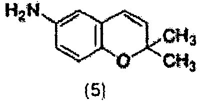 with 3-penten-2-one for cyclization to obtain 2,2,7,9-tetramethyl-2H-pyrano[2,3-g]quinoline of Formula (6): 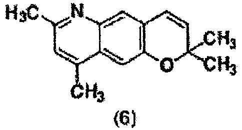
and (f) converting the obtained 2,2,7,9-tetramethyl-2H-pyrano[2,3-g]quinoline into a salt form, to obtain the salt of 2,2,7,9-tetramethyl-2H-pyrano[2,3-g]quinoline as a crystal, and neutralizing the crystal of the salt to obtain 2,2,7,9-tetramethyl-2H-pyrano[2,3-g]quinoline having higher purity.

4. The method according to claim 3, wherein, in the step (f), maleic acid is used for formation of a salt to produce 2,2,7,9-tetramethyl-2H-pyrano[2,3-g]quinoline malate of Formula (7).

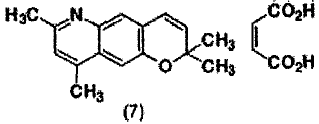

5. A method for producing 2,2,7,9-tetramethyl-2H-pyrano[2,3-g]quinoline comprising the following steps (a) to (f): (in the following formulae (1) to (4), R¹ is a C₁₋₆ alkyl group):
(a) reacting a compound of Formula (1): 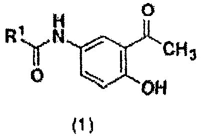 with acetone to obtain a chromanone ring derivative of Formula (2): 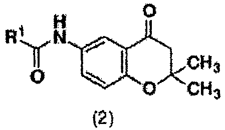
(b) reducing the obtained chromanone ring derivative to obtain an alcohol derivative of Formula (3): 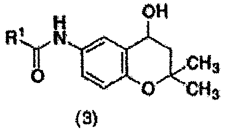
(c) dehydrating the obtained alcohol derivative to obtain a chromene ring derivative of Formula (4): 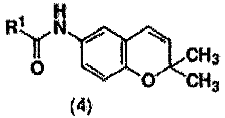
(d) hydrolyzing the obtained chromene ring derivative to obtain 2,2-dimethyl-2H-chromen-6-amine of Formula (5): 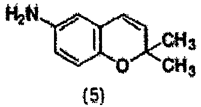
(e) reacting the obtained 2,2-dimethyl-2H-chromen-6-amine with 3-penten-2-one for cyclization, to obtain 2,2,7,9-tetramethyl-2H-pyrano[2,3-g]quinoline of Formula (6): 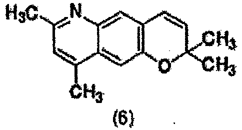 and
(f) converting the obtained 2,2,7,9-tetramethyl-2H-pyrano[2,3-g]quinoline into a salt form, to obtain the salt of 2,2,7,9-tetramethyl-2H-pyrano[2,3-g]quinoline as a crystal, and neutralizing the crystal of the salt to obtain 2,2,7,9-tetramethyl-2H-pyrano[2,3-g]quinoline having higher purity.

6. A compound of Formula (2').

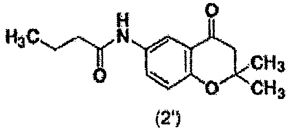

7. A compound of Formula (3').

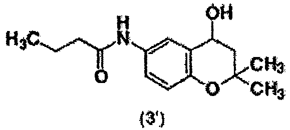

8. A compound of Formula (4').
